# EUROPEAN PATENT SPECIFICATION

(11) **EP 2 501 284 B1**
(45) Date of publication and mention of the grant of the patent: **04.01.2017**
(21) Application number: 10832049.0
(22) Date of filing: 16.11.2010
(51) Int. Cl.: A61B 5/11, A61B 5/16, A61B 5/00

(54) **METHOD FOR DIAGNOSING ADHD AND RELATED BEHAVIORAL DISORDERS**
VERFAHREN ZUR DIAGNOSTIZIERUNG VON ADHD UND ASSOZIIERTEN VERHALTENSSTÖRUNGEN
MÉTHODE DE DIAGNOSTIC DU TROUBLE D'HYPERACTIVITÉ AVEC DÉFICIT DE L'ATTENTION (THADA) ET DE TROUBLES DU COMPORTEMENT ASSOCIÉS

(30) Priority: 18.11.2009 US 262340 P
(43) Date of publication of application: 26.09.2012
(73) Proprietor: The McLean Hospital Corporation, Belmont, MA 02478 (US)
(72) Inventor: TEICHER, Martin, Rye NH 03870 (US); OHASHI, Kyoko, Arlington MA 02474 (US)
(74) Representative: Roberts, Mark Peter
(86) International application number: PCT/US2010/056805
(87) International publication number: WO 2011/062890

(56) References cited:
- US-A1- 2001 029 319
- US-A1- 2003 028 081
- US-A1- 2008 021 351
- HANISCH C ET AL: "Oculomotor inhibition in children with and without attention-deficit hyperactivity disorder (ADHD)", JOURNAL OF NEURAL TRANSMISSION ; BASIC NEUROSCIENCES, GENETICS AND IMMUNOLOGY, PARKINSON'S DISEASE AND ALLIED CONDITIONS, ALZHEIMER'S DISEASE AND ADOLESCENT PSYCHIATRY RELATED DISORDERS, BIOLOGICAL PSYCHIATRY, BIOLOGICAL CHILD AND ADOLESCENT PSYCHIAT, vol. 113, no. 5, 1 May 2006 (2006-05-01), pages 671-684, XP019378175, ISSN: 1435-1463, DOI: 10.1007/S00702-005-0344-Y

## Description

The invention relates to methods and systems for the diagnosis of ADHD and related disorders.

Attention-Deficit/Hyperactivity Disorder (ADHD) is one of the most common neuropsychiatric disorders of childhood, conservatively estimated to affect 3%-9% of school-age children (Anderson at al., Archives of General Psychiatry 44:69 (1987); Bird et al., Archives of General Psychiatry 45:1120 (1988); and Szatmari et al., J Child Psychol Psychiatry 30:219 (1989)). ADHD is characterized by a triad of symptoms involving deficits in attention, impulse control, and hyperactivity (Barkley RA. J Dev Behav Pediatr. 11:343 (1990); and Tryon WW. Behav Mod. 17:371 (1993)).

Hyperactivity is a discernible sign (rather than symptom) of the disorder, and a variety of objective instruments have been used over the last 40 years to confirm its presence (Tryon WW. Behav Mod. 17:371 (1993); Teicher MH. Harv Rev Psychiatry 3:18 (1995)). The most successful early studies used actigraphs and found that children with ADHD were about 25% to 30% more active then normal controls, particularly during academic classroom activities (Porrino et al., Arch Gen Psychiatry 40:681 (1983)), or during performance of laboratory-based attention tasks (Halperin et al., J Am Acad Child Adolesc Psychiatry 31:190 (1992); and Halperin et al., J Am Acad Child Adolesc Psychiatry 32:1038 (1993)). Increased motor activity was also present during sleep (Porrino et al., Arch Gen Psychiatry 40:681 (1983)). However, children
with ADHD were no more active than normal controls when allowed to play. These studies provide the fundamental insight that hyperactivity in children with ADHD may manifest most clearly as a diminished ability to inhibit activity to low levels (e.g., to sit or lie still) (Teicher MH. Harv Rev Psychiatry 3:18 (1995); and Porrino et al., Arch Gen Psychiatry 40:681 (1983)).

A more detailed understanding of seated hyperactivity in ADHD was obtained using a high resolution infrared motion analysis system that tracked head and body position during a computerized attention task (Teicher et al., J Am Acad Child Adolesc Psychiatry 35:334 (1996)). This study revealed that children with ADHD spent 66% less time immobile than normal, moved their head 3.4 times as far, covered a 3.8 times greater area, and had a movement pattern that was more linear and less spatially complex.

US 2008/0021351 A1 discloses a method for diagnosing ADHD and related behavioral disorders.

"Oculomotor inhibition in children with and without attention-deficit hyperactivity disorder (ADHD)", J Neural Transm (2006) 113: 671-684, by C. Hanisch *et al.* discloses that control children showed a higher percentage of successfully inhibited saccades than ADHD patients.

Nevertheless, there is still a great deal that remains to be learned about the hyperactivity of children with ADHD, and this knowledge may provide fundamental insight into the nature of the disorder. For example, an impaired ability to inhibit activity can result from at least three different mechanisms. First, a child with ADHD may not be able, even briefly, to inhibit their activity to the same low level as controls. Second, they may be able to briefly inhibit their activity to very low levels, but may not be able to sustain this degree of suppression for long. This may culminate in frequent spikes or bursts of activity. Third, they may be able to inhibit as well, and for as long, but when inhibitory control falters, and break-through movements occur, they may be of much greater amplitude.

Understanding the precise nature of seated hyperactivity is important as it is possible that some children with ADHD primarily suffer from a deficit in inhibitory control, while others have an impaired regulation of posture or position. These may represent distinct endophenotypes, with different neurobiology, genetics and treatment response.

There is a need for reliable, inexpensive, and easy to use methods for diagnosing ADHD and related disorders.

According to the present invention, which is defined in the claims, there is provided a system for diagnosing a disorder selected from ADHD and related disorders in a subject according to the independent system claim. According to the present invention, there is also provided a method of diagnosing ADHD or a related disorder in a subject according to the independent method claim. The methods and systems of the invention may also be used to ascertain how much benefit an individual would derive from a particular therapy.

The disorder may be ADD, ADHD, or Hyperkinetic Disorder, or a specific subtype of ADD, ADHD, or Hyperkinetic Disorder.

There is disclosed a method of diagnosing ADHD or a related disorder in a subject including the steps of: (i) providing motor activity data having been collected by using a motion analysis device to record movements of the subject; (ii) analyzing the motor activity data to calculate a value for a measure of positional stability in the subject; and (iii) on the basis of the value, determining whether the subject has the disorder.

There is disclosed a method of diagnosing ADHD or a related disorder in a subject including the steps of: (i) using a motion analysis device to record movements of the subject and produce motor activity data; and (ii) transmitting the data to a computer for analysis, wherein the analysis includes analyzing the motor activity data to calculate a value for a measure of positional stability in the subject, and on the basis of the value, determining whether the subject has the disorder.

The measure of positional stability may be calculated from the predictability, persistence, or stability of the motor activity data (e.g., the measure of positional stability can be calculated using the approximate entropy, maximal Lyapunov exponent, or spectral exponent of the motor activity data).

The motor activity data may include the recorded movements of the subject's head.

There is disclosed a method of diagnosing ADHD or a related disorder in a subject including the steps of: (i) providing motor activity data having been collected by using a motion analysis device to record movements of the subject; (ii) analyzing the motor activity data to calculate the spike area in the motor activity; and (iii) on the basis of the spike area, determining whether the subject has the disorder.

There is disclosed a method of diagnosing ADHD or a related disorder in a subject including the steps of: (i) using a motion analysis device to record movements of the subject and produce motor activity data; and (ii) transmitting the data to a computer for analysis, wherein the analysis includes analyzing the motor activity data to calculate the spike area in the motor activity, and on the basis of the spike area, determining whether the subject has the disorder.

The method may further include collecting data from an attentional test on the subject while recording the movements.

The motion analysis device may include an infrared motion analysis system for tracking the movements
of the subject's head, leg, elbow, shoulder, hand, or foot using a camera. Desirably, the motion analysis device includes an infrared motion analysis system for tracking the movements of the subject's head using a camera.

There is disclosed a method for assessing the efficacy of a medicament for the treatment of ADHD or a related disorder in a subject diagnosed with the disorder, the method including:
(a) providing motor activity data having been collected by using a motion analysis device to record movements of the subject following administration of the medicament to the subject; (b) analyzing the motor activity data to calculate a value for a measure of inhibitory control in the subject; and (c) on the basis of the value, determining whether the symptoms of the disorder are ameliorated by the medicament.

There is disclosed a method for assessing the efficacy of a medicament for the treatment of ADHD or a related disorder in a subject diagnosed with the disorder, the method including:
(a) administering the medicament to the subject; (b) using a motion analysis device to record movements of the subject and produce motor activity data; and (c) transmitting the data to a computer for analysis, wherein the analysis includes analyzing the motor activity data to calculate a value for a measure of inhibitory control in the subject, and on the basis of the value, determining whether the symptoms of the disorder are ameliorated by the medicament.

The measure of inhibitory control may be calculated using the spike amplitude or baseline amplitude of the motor activity data.

There is disclosed a method for assessing the efficacy of a medicament for the treatment of ADHD or a related disorder in a subject diagnosed with the disorder, the method including:
(a) providing motor activity data having been collected by using a motion analysis device to record movements of the subject following administration of the medicament to the subject; (b) analyzing the motor activity data to calculate a value for a measure
   of positional stability in the subject; and (c) on the basis of the value, determining whether the symptoms of the disorder are ameliorated by the medicament.

There is disclosed a method for assessing the efficacy of a medicament for the treatment of ADHD or a related disorder in a subject diagnosed with the disorder, the method including:
(a) administering the medicament to the subject; (b) using a motion analysis device to record movements of the subject and produce motor activity data; and (c) transmitting the data to a computer for analysis, wherein the analysis includes analyzing the motor activity data to calculate a value for a measure of positional stability in the subject, and on the basis of the value, determining whether the symptoms of the disorder are ameliorated by the medicament.

The measure of positional stability is calculated from the predictability, persistence, or stability of the motor activity data (e.g., the measure of positional stability can be calculated using the approximate entropy, maximal Lyapunov exponent, or spectral exponent of the motor activity data).

The motor activity data may include the recorded movements of the subject's head.

There is disclosed a method for assessing the efficacy of a medicament for the treatment of ADHD or a related disorder in a subject diagnosed with the disorder, the method including:
(a) providing motor activity data having been collected by using a motion analysis device to record movements of the subject following administration of the medicament to the subject; (b) analyzing the motor activity data to calculate the spike area in the motor activity; and (c) on the basis of the spike area, determining whether the symptoms of the disorder are ameliorated by the medicament.

There is disclosed a method for assessing the efficacy of a medicament for the treatment of ADHD or a related disorder in a subject diagnosed with the disorder, the method including:
(a) administering the medicament to the subject; (b) using a motion analysis device to record movements of the subject and produce motor activity data; and (c) transmitting the data to a computer for analysis, wherein the analysis includes analyzing the motor activity data to calculate the spike area in the motor activity, and on the basis of the spike area, determining whether the symptoms of the disorder are ameliorated by the medicament.

The disorder may be ADD, ADHD, or Hyperkinetic Disorder, or a specific subtype of ADD, ADHD, or Hyperkinetic Disorder.

The medicament may be a stimulant.

There is disclosed a method of diagnosing ADHD or a related disorder in a medicated subject including the steps of: (a) providing motor activity data having been collected by using a motion analysis device to record movements of the medicated subject; (b) analyzing the motor activity data to calculate a value for a measure of positional stability in the medicated subject; and (c) on the basis of the value, determining whether the medicated subject has the disorder.

There is disclosed a method of diagnosing ADHD or a related disorder in a medicated subject including the steps of: (a) using a motion analysis device to record movements of the medicated subject and produce motor activity data; and (b) transmitting the data to a computer for analysis, wherein the analysis includes analyzing the motor activity data to calculate a value for a measure of positional stability in the medicated subject, and on the basis of the value, determining whether the medicated subject has the disorder.

The measure of positional stability may be calculated from the predictability, persistence, or stability of the motor activity data. The measure of positional stability may be calculated using the maximal Lyapunov exponent of the motor activity data.

The medicated subject may be medicated with a stimulant e.g., methylphenidate or an amphetamine) or a nonstimulant (e.g., a tricyclic antidepressant, atomoxetine, bupropion, modafinil, guanfacine, or clonidine).

The metrics described herein can be very difficult for a normal subject to fake in the methods and systems of the invention. There is disclosed a method of identifying a subject faking the symptoms of ADHD or a related disorder including the steps of: (a) providing motor activity data having been collected by using a motion analysis device to record movements of the subject; (b) analyzing the motor activity data to calculate a value for a measure of positional stability in the subject; and (c) on the basis of the value, determining whether the subject is faking the symptoms of the disorder.

There is disclosed a method of identifying a subject faking the symptoms of ADHD or a related disorder including the steps of: (a) using a motion analysis device to record movements of the subject and produce motor activity data; and (b) transmitting the data to a computer for analysis, wherein the analysis includes analyzing the motor activity data to calculate a value for a measure of positional stability in the subject, and on the basis of the value, determining whether the subject is faking the symptoms of the disorder.

The measure of positional stability can be calculated from the predictability, persistence, or stability of the motor activity data. The measure of positional stability may be calculated using the maximal Lyapunov exponent of the motor activity data.

The method may further include collecting data from an attentional test on the subject while recording the movements.

The motion analysis device may include an infrared motion analysis system for tracking the movements of the subject's head, leg, elbow, shoulder, hand, or foot using a camera. Desirably, the motion analysis device includes an infrared motion analysis system for tracking the movements of the subject's head using a camera.

In any of the above methods in which spikes in the subject's movement are monitored, cluster analysis can be used to identify spikes in the movements.

There is disclosed a system for diagnosing a disorder selected from ADHD and related disorders in a subject including: (i) an input component configured to receive information including motor activity data obtained by monitoring the movements of the subject; and (ii) a processor provided with a computer program for calculating the spike amplitude of the motor activity.

There is disclosed a system for diagnosing a disorder selected from ADHD and related disorders in a subject including: (i) an input component configured to receive information including motor activity data obtained by monitoring the movements of the subject; and (ii) a processor provided with a computer program for calculating the baseline amplitude of the motor activity.

There is disclosed a system for diagnosing a disorder selected from ADHD and related disorders in a subject including: (i) an input component configured to receive information including motor activity data obtained by monitoring the movements of the subject; and (ii) a processor provided with a computer program for calculating the spike area of the motor activity.

There is disclosed a system for diagnosing a disorder selected from ADHD and related disorders in a subject including: (i) an input component configured to receive information including motor activity data obtained by monitoring the movements of the subject; and (ii) a processor provided with a computer program for calculating the approximate entropy of the motor activity.

There is disclosed a system for diagnosing a disorder selected from ADHD and related disorders in a subject including: (i) an input component configured to receive information including motor activity data obtained by monitoring the movements of the subject; and (ii) a processor provided with a computer program for calculating the maximal Lyapunov exponent of the motor activity.

There is disclosed a system for diagnosing a disorder selected from ADHD and related disorders in a subject including: (i) an input component configured to receive information including motor activity data obtained by monitoring the movements of the subject; and (ii) a processor provided with a computer program for calculating the spectral exponent of the motor activity.

In any of the above systems, the system can further include a processor provided with a computer program for generating a report of the analysis of the motor activity data.

As used herein, "ADHD or a related disorder" refers to disorders characterized by developmentally inappropriate degrees of inattention, overactivity, and impulsivity, such as Attention Deficit Hyperactivity Disorder - combined subtype, Attention Deficit Hyperactivity Disorder - predominantly hyperactive-impulsive subtype, Attention Deficit Hyperactivity Disorder - predominantly inattentive subtype, Attention Deficit Disorder with or without hyperactivity, Hyperkinetic Disorder, oppositional defiant disorder and conduct disorder. Attention Deficit Hyperactivity Disorder is a disorder characterized by inattention, impulsiveness, and hyperactivity. This disorder can impair social function, learning and/or development and is therefore now recognized as a serious problem. It is further recognized that many children with ADHD go on to develop other comorbid conditions or social problems in adulthood. In clinical terms ADHD is diagnosed if any one of the three main clinical features, inattention, over-activity, and impulsiveness, persists in two or more situations, e.g. in both a home and school environment (American Psychiatric Association. Diagnostic and Statistical Manual of Mental Disorders, Fourth Edition (DSM-IV) Washington D.C.; American Psychiatric Association, 1994). A diagnosis of Hyperkinetic Disorder is made only if all three of the main clinical features (inattention, over-activity and impulsiveness) have been present from an early age, persist in more than one situation (e.g. home and school) and impair function (The ICD-10 Classification of Mental and Behavioural Disorders: Diagnostic Criteria for Research. Geneva: World Health Organisation, 1993: 155-7).

As used herein, the term "spike" refers to a burst in the rate of movement in a subject that exceeds the baseline rate of movement, or a threshold amplitude (e.g., 1 mm/sec). Spikes can be measured across the test period using any suitable time scale from milliseconds to minutes, preferably seconds, and amplitudes set to scale from a maximum value in the reference range to a suitable minimum of about 2 x the baseline. For example, the spike amplitude can be in the range of 1 mm/sec to 8 mm/sec. The 'spike' continues until the movement falls back below the threshold amplitude level, ending the spike. Optionally, the spike doesn't end unless the movement remains below the threshold amplitude level for a set minimum amount of time (e.g., 240 msec to 1 sec). Such analysis can be accomplished using a variety of techniques, such as wavelet analysis, or by requiring the spike train to remain below threshold for a minimum time period. Alternatively, spikes can be identified using cluster analysis (see Example 1).

As used herein, the term "spike area" refers to the area of a spike and can be calculated, for example, by multiplying the spike amplitude by the duration of the spike.

As used herein, the term "baseline amplitude" refers to the average rate of movement in a subject for the data which is not part of a spike (i.e., the average of the non-spike movement data).

As used herein, the term "measure of inhibitory control" refers to a measure of the ability of a subject to inhibit activity in either degree and/or duration of suppression. The measure of inhibitory control is extracted from the motor activity data of a subject. Measures of inhibitory control include the baseline amplitude and spike amplitude of the motor activity data.

As used herein, the term "measure of positional stability" refers to a measure of the predictability, persistence, and/or stability in the motor activity data of a subject (i.e., the marker stability). To evaluate the predictability of the subject's head movements, the Approximate Entropy (ApEn) motion data was calculated. This method is related to Kolmogorov entropy and is revised to be applicable to finite, noisy time series of physiological and clinical data (Pincus SM., Proc Natl Acad Sci USA. 88:2297 (1991); Pincus et al., Am J Physiol. 266:H1643 (1994)). The Matlab (The Mathworks) code for Approximate Entropy was downloaded and implemented. Time series that are highly irregular and unpredictable will have large ApEn values and those that are more predictable consisting more repetitive patterns of fluctuation will have smaller ApEn values. For the ApEn calculation, two parameters must be assigned. One is the filter factor r and another is the length of the run m. ApEn measures the likelihood of runs with repetitive patterns (m) that are close (distance within r) for a certain observation m, and remain close to the next incremented observation m+1. The ApEn will be large for irregular and unpredictable data when it is unlikely that the observations will remain close. On the other hand, ApEn will be small for highly regular data when it is very likely that the observations will remain close. The persistency of the head movements of subjects was determined by calculating the spectral exponent b. To estimate the spectral exponent, coarse graining spectral analysis (CGSA; Yamamoto et al., Physica D 68:250 (1993)) was used. This method has an advantage for many biological signals, which consists harmonic or periodic components. In our head movement data there was a strong periodicity of 2 seconds, which corresponds to the inter-stimulus-interval. CGSA has the ability to extract the harmonic components from the time series, which appear as sharp peaks in the power spectra, to enable more accurate estimation of the spectral exponent. Unpredictable changes over time t of a quantity V is known as noise V(t). The spectral density of V(t), Sᵥ(f), gives an estimate of the mean square fluctuations of the quantity at a frequency f. By plotting log Sᵥ(f) as a function of log f, a slope can be calculated, and this slope can be interpreted as having a functional form 1/f^{β}, where β, a spectral exponent, is the negative slope of this relation. As β increases the spatial correlation in the time series also increases. This behavior indicates a gradual increase of the memory, and thus a gradual reduction of complexity in the underlying dynamics. Thus, β is a measure of the persistency of the motion of a subject (i.e., continuing in the same direction more often than if the motion was completely random). When the time series has a larger spectral exponent, it is characterized with more persistency. On the other hand, when the time series is less persistent, it will have a smaller spectral exponent. A Maximal Lyapunov exponent (MLE) was used as a measure of local stability. This technique in nonlinear dynamics examines the dynamical characteristics of the time series by embedding them into state space. The Lyapunov exponent quantifies the rate of separation in the course of time of very close points in the state space and quantifies the effect of perturbations in a dynamical system as well as its dependence on initial conditions. For our data, Lyapunov exponent can be interpreted as the stability of the head movements of the subject. In order to estimate MLE accurately, embedding parameters, dimensions (m) and time delay (r) were chosen referring to the time delayed mutual information and false nearest neighborhood method respectively. To verify whether the time series is deterministic in nature, method of "surrogation" was applied, which compares surrogate data to the original data. Surrogate data are generated from the original data by randomization. This randomization removes its deterministic structure but is done carefully to conserve the mean, variance and the power spectra of the original data. By comparing the MLE of surrogate data to the original data, we could see if the original data were randomly derived, i.e., the surrogate data has the same MLE compared to the original data, or if the original data is deterministic in nature, i.e, MLE for surrogate and the original data are significantly different. Alternative methods for measuring the predictability, persistence, and/or stability of a data set are known in the art and can be used in the methods and systems of the invention.

Other features and advantages of the invention will be apparent from the following detailed description, the drawings, and the claims.

### Brief Description of the Drawings

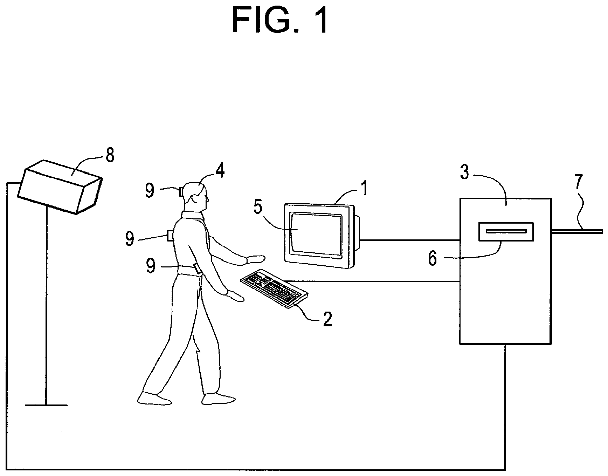

Figure 1 depicts a system for performing a method of the invention. The system includes a motion analysis device **8** that is connected to the computer **3** and positioned so as to record the movements of the subject **4**.

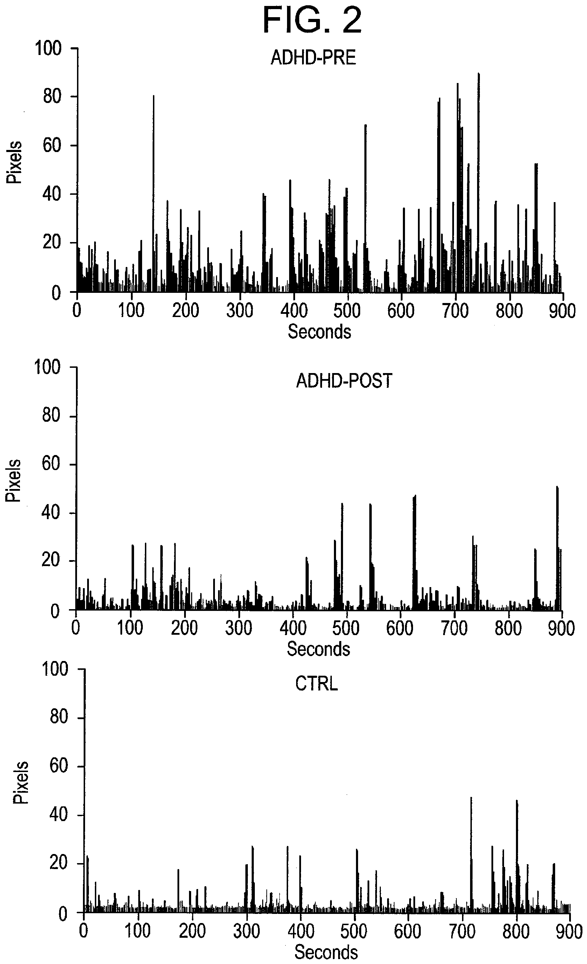

Figure 2 is three graphs depicting the head position time series indicating absolute change in pixel distance between successive measures captured at 50 Hz during a 15-minute cognitive control task for a typical subject with ADHD before (a) and after (b) methylphenidate, and a representative subject from the contrast group (c). Red and blue regions indicate spike and baseline activity respectively. The data is averaged each second for visual purposes.

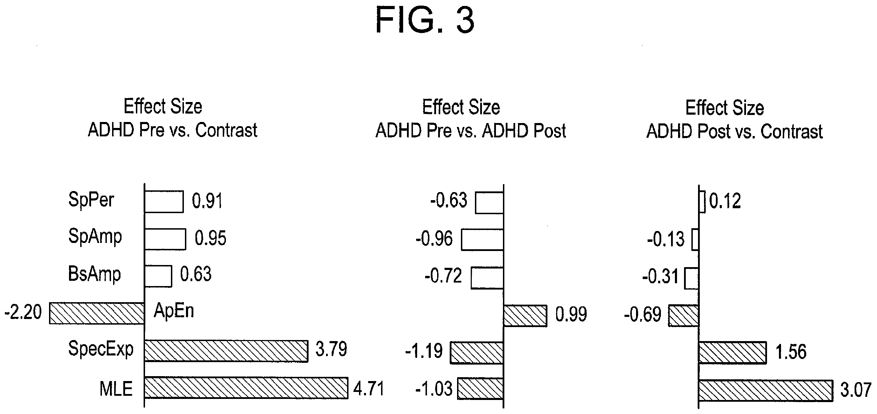

Figure 3 is three graphs depicting the Cohen's d' effect size for percentage of spike (SpPer), spike amplitude (SpAmp), baseline amplitude (BsAmp), approximate entropy (ApEn), spectral exponent (SpecExp) and maximal Lyapunov exponent (MLE) between ADHD groups prior and post medication and contrast group.

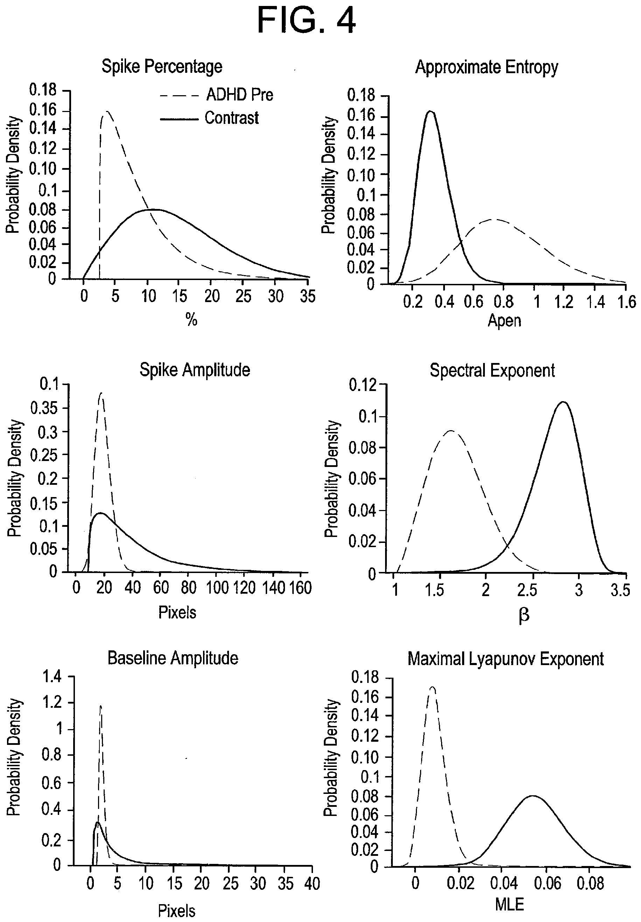

Figure 4 is a series of graphs depicting the probability density curves for ADHD subjects PRE medication versus contrast controls. The distribution of scores for all subjects in a group were fit to normal, log-normal, gamma or Weibull distributions to reveal theoretical degree of overlap for the populations, and the shapes of the curves. The figure shows the best fitting probability density curve for each group. Goodness of fit for each series of quasi-normal curves was evaluated by Kolmogorov-Smirnov tests.

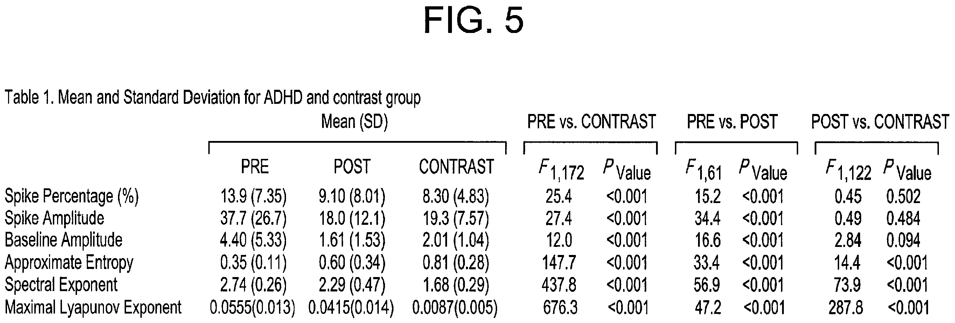

Figure 5 is a table of mean values for subjects with ADHD and contrast controls (CONTRAST = contrast control; PRE = subjects with ADHD before methylphenidate administration; and POST = subjects with ADHD 120 minutes after methylphenidate administration). On average, ADHD subjects had baseline and spike amplitudes that were 2.2- and 2.0-fold greater than controls, respectively. The degree of inhibitory control was markedly enhanced by administration of methylphenidate as (i) baseline and spike amplitudes were reduced by 63% and 52%, respectively, yielding effect sizes of about 0.7 to 1.0; and (ii) the number of spikes was reduced by 35%. The net result of methylphenidate administration was that baseline and spike amplitudes, and spike numbers, were suppressed to at or below contrast group levels.

We have discovered that children with ADHD had deficits in both inhibitory control and positional stability. Deficits in positional stability were universal, affecting virtually all of the ADHD subjects and none of the contrast controls. Inhibitory deficits affected both the degree and duration of suppression, but there was more overlap between ADHD and contrast controls on these parameters. MPH dramatically enhanced the degree of inhibition, enabling children with ADHD to suppress activity to at least the same low levels as contrast subjects. MPH only partially attenuated deficits in maintenance of positional stability, which remained quite impaired. Hence, ADHD hyperactivity appears to be characterized by at least two abnormalities that differ significantly in degree of correctability by methylphenidate.

The invention exploits the relationship between measures of inhibitory control and positional stability, and ADHD to provide methods and systems for the diagnosis of ADHD and related disorders and to provide methods and systems for ascertaining how much benefit an individual would derive from a particular therapeutic regimen (see Example 1).

### Systems

An embodiment of a system for performing a method of the invention is shown in Figure 1. The system includes a motion analysis device **8** that is connected to the computer **3** and positioned so as to record the movements of the subject **4**. Any video camera or other motion-sensing device capable of detecting the movements of the subject **4** can be used. For instance, the motion analysis device **8** can be an infrared motion analysis system that includes a high-resolution CCD infrared video camera, an infrared strobe, and a video processor that provides hardware analysis of the video signal and outputs data to the computer **3**. Such infrared motion analysis systems are known in the art, and are specifically designed to detect and record the precise vertical and horizontal position of small, light-weight infrared reflective markers **9**. These markers **9** are attached to the subject **4** at various points, such as the head, shoulders, and elbows. As the subject **4** moves these portions of his or her body, the IR motion analysis system detects changes in the positions of the markers **9** and relays this information to the computer **3**. Successive marker coordinates can be stored in the computer **3** and analyzed.

The computer **3** can be a stand-alone personal computer, preferably with high computational capacity microprocessors. Alternatively, a minicomputer or mainframe computer can be used. The computer **3** can have a disc drive **6** into which the software that analyzes the subject's input's and/or movement patterns is loaded. In a preferred embodiment, the computer **3** has a connection **7** to a network of computers, such as a global computer network. This allows the computer **3** to exchange data with other computers connected to the network. In other preferred embodiments, the computer network is a local area network, a wide area network, an intranet, or an extranet. Thus, a subject may be tested not only in a clinical setting, but also at a remote location, such as the home, school, or workplace, thereby eliminating the inconvenience of traveling long distances for testing.

The system may also include a monitor **1** that is a capable of displaying visual images on a screen **5**. The monitor 1 is attached to a computer 3 and is positioned in proximity to a subject **4**, so that the subject **4** may view the images displayed on the monitor screen **5**. The computer **3** can be programmed to display a desired sequence of images, to which the subject **4** is instructed to respond by activating an input device **2** that is also attached to the computer **3** and is controllable by the subject **4**. The input device **2** can be, for example, a standard computer keyboard, a hand-held plunger switch, or a large, easy-to-hit switch several (2-3) inches in length. When activated, the input device **2** sends the subject's inputs to the computer **3** which stores and analyzes the incidents of device activation.

At the end of the testing, the recorded data (e.g., key press information and movement information) can be processed by a local computer or transmitted over a computer network to a central station for processing. A report can be generated at the testing site, or at the site of remote processing. Such a report may be in a paper form, electronic form, or stored in a database as part of the subject's medical records. The report can include one or more of the following: (i) the unmedicated and/or medicated results for one or more measures of inhibitory control or positional stability, or a composite thereof, for a test subject; (ii) the results obtained for a subject and the range of results observed for normal subjects given the subject's gender, age, and/or grade; (iii) the unmedicated results for approximate entropy, maximal Lyapunov exponent, spectral exponent, baseline amplitude, spike amplitude, spike area, or a composite value comprising one or more of these measures, for a test subject; (iv) the medicated results for approximate entropy, maximal Lyapunov exponent, spectral exponent, baseline amplitude, spike amplitude, spike area, or a composite value comprising one or more of these measures, for a test subject; and (v) the results for approximate entropy, maximal Lyapunov exponent, spectral exponent, baseline amplitude, spike amplitude, spike area, or a composite value comprising one or more of these measures, for a test subject along with a warning that the subject may be faking the symptoms of disease (e.g., to gain access to stimulant drugs used to treat the disease).

### Motion Detection System

A motion detection system is used to track the movement of the head and/or lower extremities of the individual being tested. Any video camera or other motion-sensing device capable of detecting the movements of the test subject can be used. For example, the motion analysis device can be an infrared motion analysis system (e.g., Qualisys, Glastonbury, CT) that includes a high-resolution CCD infrared video camera, an infrared strobe, and a video processor that provides hardware analysis of the video signal and outputs data to a computer. Such infrared motion analysis systems are known in the art, and are specifically designed to detect and record the precise vertical and horizontal position of small, light-weight infrared reflective markers. These markers are attached to the subject at various points, such as the head, shoulders, arms, legs, and feet. As the subject moves these portions of his or her body, the IR motion analysis system detects changes in the positions of the markers and relays this information to a computer. Successive marker coordinates can be stored in the computer and analyzed. Desirably, the camera is positioned in front of the subject, who is preferably in a seated position. The camera is also desirably positioned in such a manner that it can capture movements of the reflective markers in three dimensions, including movements towards and away from the display device. The motion analysis device can also include a second camera that can be used in combination with the first camera to better differentiate three dimensional movement. Adults with ADHD or related disorders can manifest hyperactivity solely through excess movement of their lower extremities while seated. Therefore, the first camera can be used to track the movement of the subject's legs and/or feet or a second camera can be used to track the movement of the subject's lower extremities while the first camera tracks upper body movements. Alternatively, visible light and standard video camera are used to measure the movement of a subject, or an accelerometer is used.

Movement patterns can be analyzed using procedures described by Teicher et al., J. Am. Acad. Child Adolsec. Psychiatry 35:334 (1996), which are based on the concept of microevents. A new microevent begins whenever the marker moves more than a predetermined distance from the location of the previous microevent, and is defined by its position and duration.

A variety of statistical techniques can be used in connection with the methods and systems of the invention. For example, the movement time series can be analyzed using a mathematical techniques such as Fourier Transform, Wigner-Wille Transform, or wavelet analysis to decompose time series from time domain into frequency domain.

### Attentional Testing

The subject is, desirably, engaged in an attentional test while the motor activity of the subject is monitored. The attentional testing includes a cognitive control task, such as a continuous performance test (CPT), the results of which are diagnostic of physiological response to medication. For example, a subject's visual attention can be tested by displaying a series of visual stimuli, to which the subject is instructed to respond. Typically, the stimuli are of two types, and the subject is instructed to respond to only one of them. Data are collected for each stimulus presented including the type of stimulus, whether or not the subject responded, and if so, how long the subject took to respond. The continuous performance attention test has been in use since the mid 50's (Rosvold et al., J. Consulting and Clinical Psychology 20:343 (1956)), with computerized versions available in the 1970's (Greenberg, Psychopharmacol. Bull. 23:279 (1987)).

The CPT results can include measuring errors of commission, errors of omission, and mean correct reaction time with standard deviation. More sophisticated CPT measures, derived from signal detection theory can include a calculation of stimulus sensitivity (d') (see, for example, Nuechterlein, J. Abnorm. Psychol. 92:4 (1983)).

Analysis of the CPT results can also include assessing the pattern or fluctuation in attentional states by a subject during a test period. This approach is described in U.S. Patent No. 6,685,652.

The methods of the invention may be used alone, together, or in conjunction with other well-known psychological tests for determining attention or reaction time. Testing of the subject's performance may be conducted with or without providing corrective feedback to the subject during performance of the CPT.

### Therapy and Dosing Regimens

The methods and systems of the invention can provide information on the efficacy of any particular therapy in an individual. For example, using the methods of the invention it can be possible to determine how a subject would respond to any of the different long acting stimulant preparations (e.g., Concerta™ 18, 36, and 54 mg; Metadate CD™ 20, 40, 60 mg; Ritalin-LA™ 10-60 mg) or combinations, such as Ritalin-LA™ 40 mg taken at 8 am and
Ritalin™ immediate release 15 mg taken at 4 PM. These assessments are made based upon the degree of improvement in a subject's motor activity and, optionally, performance on CPT testing.

If a test subject fails to show substantial benefits on one class of stimulants (i.e., methylphenidate versus amphetamine derivatives, such as dextroamphetamine or Adderall), the subject can be tested on a separate day on a drug from the other class of stimulants. Clinical research has shown that patients with ADHD often respond better to one class of stimulants than another, and that a significant number of patients with ADHD will have a very beneficial response to one class of agents but will fail to respond to the other class, or will have side-effects on only one class (see, for example, Elia et al., Psychiatry Res. 36:141 (1991)).

Alternatively, the methods and systems of the invention allow for the diagnostic testing of medicated subjects.

Both stimulant and non-stimulant medicaments can be used in the methods of the invention.

### Stimulant Medicaments

Central nervous system stimulants, such as MPH, are used in the treatment of Attention Deficit Disorder ("ADD"), a commonly diagnosed nervous system illness in children that is characterized by both distractability and impulsivity, Attention Deficit Hyperactivity Disorder ("ADHD"), in which symptoms of hyperactivity are present along with the symptoms of ADD, and can also decrease symptoms related to co-existing conditions, such as Oppositional Defiant Disorder. Stimulants are also used in the symptomatic treatment of narcolepsy, depression, and the cognitive decline associated with Acquired Immunodeficiency Syndrome ("AIDS") or AIDS-related conditions, as well as for mood elevation, particularly in terminally ill patients with diseases such as cancer.

### Immediate Release Methylphenidate Preparations

Immediate release (IR) methylphenidate comes in brand (Ritalin) and generic (methylphenidate) formulas. IR methylphenidate begins working almost immediately (within about 20 to 30 minutes) and lasts 3 to 4 hours. The scored tablets come in 5, 10, and 20 mg scored formulations. The maximum recommended daily dose is 60 mg. Methylphenidate administered three times a day dosing was found to be more effective that twice a day dosing in the MTA study.

Focalin is the d-isomer of methylphenidate, the active isomer in regular methylphenidate which is a racemic mixture of both d and 1 isomers. Focalin is twice as potent as methylphenidate, e.g. 2.5 mg of Focalin has the same therapeutic benefit as 5.0 mg of Ritalin. Focalin begins working immediately and lasts 3 to 4 hours. The recommended starting dose for new patients is 2.5 mg twice daily. Focalin tablets come in 2.5, 5 and 10 mg formulations. The maximum recommended daily dose is 20 mg (10 mg twice daily).

### Sustained Release Methylphenidate Formulations

### Concerta^{™}

Concerta^{™} has been available since August 2000. Concerta^{™} is a capsular version of methylphenidate. IR methylphenidate coats the surface of the capsule and an OROS^{™} delivery system uses osmotic pressure to pump methylphenidate out of the capsule over the course of the day. Only 22% of the medication is released upon ingestion; the delivery system pumps the remaining 78% of the medication out over 8 to 12 hours. Concerta^{™} lasts up to 12 hours, providing smooth control without school dosing, and has not associated in the literature with a higher incidence of rebound or insomnia. Concerta^{™} is currently available in 18 mg, 27 mg, 36 mg, and 54 mg coated capsules that may not be broken or chewed because of the presence of the pump inside the capsule. The recommended maximum daily dose is 54 mg.

### Metadate CD^{™}

Metadate CD^{™} was approved in March 2001 by the FDA as an extended-release methylphenidate capsule. This medication uses a unique method of controlled drug delivery called Diffucaps^{™}. This system uses beads inside the capsule that are released in two main "waves". Approximately 30% of the dose is released immediately and 70% of the dose is available for extended release. The first peak plasma level is achieved about 1.5 hours after dose and the second peak plasma level is reached about 4.5 hours after dosing. Metadate CD^{™} comes 20 mg capsules. The maximum recommended daily dose is 60 mg.

### Metadate ER^{™}

Metadate ER^{™}, a form of methylphenidate, is available as extended-release tablets of 10 and 20 mg and is more slowly but as extensively absorbed as in the regular tablets. Metadate ER^{™} tablets have a duration of action of approximately 8 hours. The maximum recommended daily dose is 60 mg.

### Methylin ER^{™}

Methylin ER^{™} was approved by the FDA in May 2000. It is available in 10 mg and 20 mg extended release tablets. It uses a dual-acting hydrophilic polymer release technology, where the release of methylphenidate is due to diffusion and erosion. Methylin ER^{™} is thought to have a duration of action of 4 to 8 hours. The maximum recommended daily dose is 60 mg.

### Ritalin SR^{™}

Ritalin-SR^{™} (sustained release formula, methylphenidate) has been available for more than a decade. This medication takes effect within an hour after administration and may last for four to eight hours, which theoretically eliminates the need for a second dose to be taken at school. The maximum recommended daily dose is 60 mg.

### Ritalin LA^{™}

Ritalin LA^{™} is an extended-release formulation of Ritalin that eliminates mid-day dosing. Ritalin LA^{™} is available in 10,20, 30 and 40 mg. Ritalin LA^{™} administers an immediate dose of methylphenidate upon consumption and a second dose approximately 4 hours later. Effects of Ritalin LA^{™} have a duration of approximately 6-8 hours. The maximum recommended daily dose is 60 mg.

### Daytrana^{™}

Daytrana^{™}, formally known as MethylPatch^{™}, is a medicinal patch marketed by Shire Pharmaceuticals and is most commonly referred to as Methylphenidate Transdermal System (MTS). Daytrana is FDA approved as a once daily treatment of pediatric patients, ages 6 to 12, with Attention Deficit Hyperactivity Disorder. Oral-based methylphenidate pharmaceuticals can be subject to first-pass hepatic metabolism, and the levo-isomer is extensively metabolized, consequently contributing nothing to the dextro-isomer's clinical value. In contrast, Daytrana^{™} is administered transdermally and avoids most first-pass hepatic metabolism. As a result, the levo-isomer accounts for a thirteenth of Daytrana's efficacy.

### Amphetamine Formulations

### Adderall^{™}

Adderall^{™} is a mixture of amphetamine salts (dextroamphetamine saccharate, dextroamphetamine sulfate, aspartate d/l-amphetamine, and sulfate d/l-amphetamine) formulated for immediate release. Adderall is marketed in unit dosage forms of 2.5 mg, 5 mg, 7.5 mg, 10 mg, 12.5 mg, 15 mg, 20 mg, 25 mg, and 30mg strengths.

### Adderall XR^{™}

Adderall XR^{™} is an extended-release formulation containing a mixture of amphetamine salts. These four amphetamine salts are reported to be metabolized at different rates and to possess diverse half lives, therefore resulting in a less dramatic onset and termination of therapeutic action; as compared to single salt amphetamine preparations. Adderall XR^{™} is marketed in unit dosage forms of 2.5 mg, 5 mg, 7.5 mg, 10 mg, 12.5 mg, 15 mg, 20 mg, 25 mg, and 30mg strengths.

### VYVANSE^{™}

VYVANSE^{™} is a therapeutically inactive prodrug, in which d-amphetamine is covalently bonded to 1-lysine, and after oral ingestion it is converted to pharmacologically active d-amphetamine. VYVANSE^{™} is currently available in dosage strengths of 20 mg, 30 mg, 40 mg, 50 mg, 60 mg, and 70 mg, each for once-daily dosing.

### Nonstimulant Medicaments

Nonstimulant medicaments, such as tricyclic antidepressants (TCAs), alpha2 agonists, bupropion, modafinil, and atomoxetine are prescribed for the treatment of attentional disorders, such as ADHD.

### Atomoxetine

Atomoxetine is the first non-stimulant drug approved for the treatment of attention-deficit hyperactivity disorder (ADHD). It is manufactured and marketed under the brand name Strattera^{™} by Eli Lilly and Company. Atomoxetine is classified as a norepinephrine reuptake inhibitor, and is approved for use in children, adolescents, and adults. Its advantage over stimulants for the treatment of ADHD is that it has less abuse potential than stimulants, is not scheduled as a controlled substance, and has proven in clinical trials to offer 24 hour coverage of symptoms associated with ADHD in adults and children. Strattera^{™} is marketed in unit dosage forms of 10 mg, 18 mg, 25 mg, 40 mg, 60 mg, 80 mg, and 100mg strengths.

### Alpha2 agonists

Alpha-2 agonists, such as clonidine and guanfacine, exert their therapeutic effects through stimulation of post-synaptic alpha-2A receptors on the dendritic spines of prefrontal cortical pyramidal cells, increasing the functional connectivity of the prefrontal cortical networks, and thus strengthening the regulation of attention and behavior. Clonidine comes in 0.1, 0.2, and 0.3 mg tablets as well as a transdermal patch. The typical daily dose is 0.2 to 0.3 mg per day in three or four divided doses. Guanfacine is given in amounts between 1 mg and 3 mg per day in three divided doses.

### Tricyclic Antidepressants

Tricyclic antidepressants have been shown to be effective in treating attention-deficit hyperactivity disorder. ADHD is thought to be caused, in part, by norepinephrine shortages in the brain's prefrontal cortex. Tricyclic antidepressants block the reuptake of norepinephrine, thus acting as norepinephrine agonists. They are commonly used in patients for whom psychostimulants (the primary medication for ADHD) are ineffective. TCAs are more effective in treating the behavioral aspects of ADHD than the cognitive deficits; they help limit hyperactivity and impulsivity but have little effect on attention. TCAs which can be used include desipramine, imipramine, protriptyline, and nortriptylinc.

### Bupropion

Bupropion (Wellbutrin^{™}) is an atypical antidepressant useful for the treatment of symptoms associated with ADHD. Bupropion is a dopamine and norepinephrine reuptake inhibitor. It is about twice as potent an inhibitor of dopamine reuptake than of norepinephrine reuptake.

### Modafinil

Modafinil has been used for the treatment of ADHD, however, modafinil's mechanism of action in ADHD is unknown. It has been proposed that rather than blocking the dopamine transporter, modafinil might activate the anterior cingulate cortex. This, in turn, might affect executive function and alertness in ADHD.

The following example is put forth so as to provide those of ordinary skill in the art with a complete disclosure and description of how the methods and compounds claimed herein are performed, made, and evaluated, and are intended to be purely exemplary of the invention and are not intended to limit the scope of what the inventors regard as their invention.

### Example 1. : Inhibitory Control and Positional Stability in ADHD and Normal Subjects.

The primary purpose of the present study was to analyze the microstructure of head movements on a millisecond time scale in order to test specific hypotheses regarding capacity of hyperactive children with ADHD performing a cognitive control task to inhibit motor activity and to maintain positional control of a head marker, reflecting the relative position of their head in relationship to the computer screen. The second goal was to ascertain whether inhibitory deficits or positional control deficits had greater power to discriminate children with ADHD from more typical children. The final goal was to evaluate the effects of methylphenidate (MPH) on measures of inhibitory control and positional stability, to ascertain if deficits in these domains were equally ameliorated by treatment.

### Methods

### Subjects

Data were analyzed from a study approved and monitored by the McLean Hospital Institutional Review Board (IRB). The ADHD sample consisted of 62 boys between 9-12 years of age who met Diagnostic and Statistical Manual of Mental Disorders, 4th edition (DSM-IV) (Diagnostic and statistical manual of mental disorders : DSM-IV. --4th ed., text revision. Washington, D.C: American Psychiatric Association; 2000) criteria for ADHD combined subtype. Subjects were recruited from the general population via newspaper advertisement for hyperactive boys who were currently or previously treated with MPH, as the protocol called from them to receive a moderately large probe dose of immediate release MPH. This was easier to justify in subjects known to tolerate MPH without untoward effects. Parental written consent and child verbal assent were obtained. Most of the children enrolled were receiving treatment with MPH (92%), and the remaining children had a previous history of treatment. Seventy-three boys went through the screening procedures, which included structured interviews for DSM-IV Axis I psychiatric diagnoses using K-SADS-E (Orvaschel H, Puig-Antich J. Schedule for Affective Disorder and Schizophrenia for School-Age Children, Epidemiologic Version, Fifth Revision. Fort Lauderdale, FL: Nova Southeastern University; 1994); and parent ratings on the Conners' Hyperactivity Index (Conners et al., J Abnorm Child Psychol. 26:257 (1998)) and Achenbach Child Behavior Checklist (CBCL) (Achenbach T. Integrative Guide to the 1991 CBCL/4-18, YSR, and TRF Profiles. Burlington, VT: University of Vermont, Department of Psychology; 1991). Children with ADHD could not have any current major mood disorder, psychosis, tic disorder, a major anxiety disorder, or mental retardation. Children with oppositional defiant disorder (ODD), or reported learning disorders, could participate. From this pool of subjects, 11 were excluded from further participation. Eight children were excluded because they had an insufficient number of symptoms to meet criteria for combined subtype. One subject was disqualified for drug use, another was disqualified for current antidepressant treatment of night terrors, and one withdrew before the probe dose. These 62 subjects with ADHD-C had a mean age of 11.0 ± 1.1 years. In this sample, there were 19 boys with comorbid ODD, 2 with current dysthymia, 4 with learning disorders, and 3 with past major depression or past anxiety disorders. Children with ADHD had an average Abbreviated Conners' Hyperactivity Index (Conners et al., J Abnorm Child Psychol. 26:257 (1998)) score of 19 (any score over 15 is indicative of hyperactivity), and mean scores of 20 and 29 for Internalizing and Externalizing Problems on the CBCL. Sixty of these subjects were previously included in a study examining fluctuations in attentional state (Teicher et al., J Child Adolesc Psychopharmacol. 14:219 (2004)), and 48 were included in a study on pharmacokinetic-pharmacodynamic response to methylphenidate (Teicher et al., J Child Adolesc Psychopharmacol. 16:416 (2006)).

For comparison, we analyzed data from a representative non-clinical contrast group. This group consisted of 62 male students, randomly selected from a sample of 1168 subjects (6 - 14 years of age) tested at local public schools using teacher ratings and a computerized attention task with infrared motion analysis (Teicher et al., J Am Acad Child Adolesc Psychiatry 35:334 (1996); Quotient Test, BioBehavioral Diagnostic Company, Cambridge, MA). This large-scale database development study was approved by the McLean Hospital IRB and the school administrators. Parents provided written consent and children gave verbal assent to participate. Subjects selected from this sample were male, and matched 1:1 with the ADHD subjects by age to the nearest month (11.0 ± 1.0 years). The selection criteria applied for this analysis was that they needed to have Conners' Teacher Ratings (Conners CK. American Journal of Psychiatry 126:884 (1969)) that were within ± 1 SD of the mean of peers their own age and gender (i.e., T score < 60), to help exclude subjects with potential ADHD.

### Protocol

Children with ADHD were tested prior to (PRE) and 120-minutes following (POST) a probe dose of 0.4 mg/kg MPH. Contrast subjects only received a single Quotient Test, which was specifically designed to provide objective measures of hyperactivity, inattention and impulsivity (Teicher et al., J Am Acad Child Adolesc Psychiatry 35:334 (1996)). Each child sat on an adjustable height chair without back support, adjusted so that they were seated comfortably with both feet on the floor with knees bent at a nearly right angle. Children were instructed to place their feet on the floor, but were able to freely move their legs during the task. The attention test was presented on a computer screen on an adjustable height school desk, set so that screen height was positioned at eye level. Subjects were instructed to press a space bar every time they saw a target. Their hand rested on the desk with fingers poised directly above the space bar. Children were instructed to press a button when they saw an eight-sided star and to not press the button when a five-sided star appeared (50% target density). Stars were presented briefly (200 msec), at random screen positions, every two seconds. During this 15-minute task a small reflective marker was worn on a headband so an infrared motion analysis system could track and record the marker's vertical and horizontal position to a resolution of 0.04 mm (Teicher et al., J Am Acad Child Adolesc Psychiatry 35:334 (1996)).

### Data analysis

Horizontal and vertical head marker positions were smoothed to filter out camera noise using a five point moving average. Distance between successive marker positions were calculated, providing a 45,000 point time series of distance moved every 20 msec throughout the 15 minute test.

As illustrated in Figure 2, the head movement time series was characterized by a very low baseline level of activity punctuated by much higher amplitude movement 'spikes'. Baseline and spikes signals were discriminated using the Max-Pass method, which is a robust technique for detecting spontaneous neural spikes embedded in background activity (Liu et al., J Neurosci Methods 153:299 (2006)). Briefly, this technique calculates a statistically optimal baseline as the line through which the signal makes the maximal number of positive and negative excursions. The median average deviation (MAD) is calculated from excursions below the line (which consists of baseline activity), and a threshold is set a certain number of MAD units above the baseline to optimally distinguish spikes from baseline activity (Liu et al., J Neurosci Methods 153:299 (2006)). A conservative 8 MAD criteria was used, given the extremely high relative amplitude of spikes. However, a 4 MAD criteria produced a nearly identical pattern of results.

Because the use of max-pass for the analysis of velocity time series is novel, we also calculated baseline and spike amplitudes using cluster analysis. This technique is frequently used in electrophysiology to classify spikes from different neurons (Lewicki MS. Network 9:R53 (1998)), and can be used to detect spikes (Inan et al., Comput Biol Med. 37:1160 (2007)). For each data set, K-means cluster analyses were performed to delineate a high amplitude cluster that contained between 1% and 10% of the total number of data points. (The analysis started with two clusters and progressed up to four clusters to meet this criteria). This 'high-amplitude low frequency of occurrence' cluster identified spikes, and provided their number and mean amplitude.

The following non-linear techniques were used to characterize the predictability, persistence and stability of the activity patterns, as abnormalities in these parameters provide the strongest evidence for postural or positional instability. Approximate Entropy (ApEn) was quantified as a measure of the predictability or regularity of head marker movements. This method is related to Kolmogorov entropy and revised to be applicable to finite, noisy biological time series (Pincus SM. Proc Natl Acad Sci USA. 88:2297 (1991); and Pincus et al., Am J Physiol. 266(4 Pt 2):H1643 (1994)). ApEn was calculated using Matlab code (Physionet, http://www.physionet.org/physiotools/ApEn/; Software for Heart Rate Variability, http://www.macalester.edu/~kaplan/hrv/doc/). Time series that are highly irregular and unpredictable will have large ApEn values. For ApEn calculation, two parameters are assigned. Wc set the length of the run m=2 and the filter factor r=20% of the series standard deviation, as recommended in a previous study (Pincus et al., Am J Physiol. 266(4 Pt 2):H1643 (1994)).

Persistence of head marker movements was estimated by the spectral exponent (β), based on the general linear relation between log-power spectral density and log-frequency observed in previous papers examining human physical activity (see Aoyagi et al., Am J Physiol Heart Circ Physiol. 278:H1035 (2000); Ohashi et al., Methods Inf Med. 43:26 (2004); Ohashi et al., Phys Rev E Stat Nonlin Soft Matter Phys. 68(6 Pt 2):065204 (2003); and Selz et al., Fractals 3:893 (1995)). The spectral exponent is the negative slope of this relation, (i.e., 1/f^{β}). Time series with large β are characterized by more persistence. Coarse graining spectral analysis (CGSA) (Yamamoto et al., Physica D. 68:250 (1993)) was used to estimate b. This method was specifically designed to separate periodic from non-periodic components of biological signals. There was a strong 2-second (0.5 Hz) periodicity in head marker movements, which corresponds to the inter-stimulus-interval. CGSA was able to extract this component to provide a more accurate estimation of b.

The maximal Lyapunov exponent (MLE) was used as a measure of local stability. This technique examines the dynamic characteristics of the time series by embedding them into state space. Lyapunov exponents quantify the rate of separation over time of very close points in the state space. Thus, MLE quantifies the effect of perturbations in a dynamical system, as well as its dependence on initial conditions (degree of deterministic chaos). Elevated MLE provides strong evidence for postural or positional instability (Ladislao et al., Med Biol Eng Comput. 45:679 (2007)). MLE was calculated using TISEAN (http://www.mpipks-dresden.mpg.de/~tisean/ TISEAN_ 2.1/index.html). Embedding parameter dimensions (m) and time delay (t) were chosen using the time delayed mutual information and false nearest neighborhood methods, respectively (Fraser et al., Phys Rev A 33:1134 (1986); and Kennel et al., Phys Rev A 45:3403 (1992)). To verify whether the time series was deterministic in nature, the method of "surrogation" was applied, which compares surrogate data to the original data (Schreiber et al., Physica D 142:346 (2000)). Surrogate data were generated from the original time series by spectrally-balanced randomization (reshuffling) which removes potentially deterministic structure from the series but preserves the mean, variance and power spectra. The MLE of the original time series is considered significant, and indicative of deterministic chaos, if it is greater than MLEs calculated for the surrogate series using the rank-order test suggested by Theiler (Theiler et al., Physica D 58:77 (1992)). For each original time series, 19 surrogate comparison series were generated. Because low pass filtering can introduce spurious Lyapunov exponents (Badii et al., Phys Rev Lett. 60:979 (1988)), MLE analyses were performed on time series preprocessed using nonlinear filtering (Ladislao et al., Med Biol Eng Comput. 45:679 (2007); and Hegger et al., Chaos 9:413 (1999)). Evidence for deterministic chaos was also verified using the correlation dimension method followed by surrogation (Sivakumar B. Journal of Hydrology 227:1 (2000)).

### Statistical Analyses

Differences between contrast controls and ADHD subjects PRE and POST administration of MPH were assessed using between-subject ANOVA. MPH effects on ADHD subjects were assessed using repeated measure ANOVA. The magnitude of differences related to diagnosis and MPH administration were indicated by Cohen's d' effect size measures. Discriminative differences between ADHD and contrast controls were calculated using Receiver Operating Characteristic (ROC) analysis (Langlotz CP. Radiology 228:3 (2003)), and distribution graphs plotted to show the theoretical degree of overlap between children with ADHD and contrast controls. The interrelationship and independence of the assessed parameters were evaluated using correlation and Principle Component analysis. Statistical tests were performed in SPSS (SPSS Inc., Chicago, IL).

### Results

Head movements were divided into baseline and spike states using Max-Pass (Liu et al., J Neurosci Methods 153:299 (2006)) (see Figure 2). On average, ADHD subjects had baseline and spike amplitudes that were 2.2- and 2.0-fold greater than controls, respectively (see Table I in Figure 5). ADHD subject also had a 68% greater number of epochs classified as spikes. Effect size differences (Cohen's d') between ADHD PRE and contrast subjects ranged from 0.6 (baseline amplitude) to 1.0 (spike amplitude) (Figure 3). Spike amplitude discriminated ADHD subjects from contrast controls with modest accuracy (ROC Area = 0.799; Figure 4).

Degree of inhibitory control was markedly enhanced by MPH. Baseline and spike amplitudes were reduced by 63% and 52%, respectively (both p's < 0.0001; Table I in Figure 5), yielding effect sizes of about 0.7 to 1.0. Number of spikes was reduced by 35%, (p < 0.0001). The net result was that baseline and spike amplitudes, and spike numbers, were suppressed to at or below contrast group levels (Figure 3).

Spike detection using cluster analysis confirmed that ADHD subjects had baseline and spike amplitudes that were 2.2- and 1.9-fold greater than controls, that spike amplitude discriminated ADHD subjects from contrast controls with moderate accuracy (ROC Area = 0.822), and that MPH reduced baseline and spike amplitudes to at or below contrast group levels.

ADHD subjects off medication and contrast controls differed dramatically in stability, predictability and persistence of head marker movements. The MLE was 6.4-fold greater in ADHD subjects. Eighty four percent (52/62) of the ADHD subjects PRE showed evidence for a non-linear deterministic component in their movement time-series based on comparison to their surrogate data set. In contrast, there was no evidence of a deterministic component in any of the contrast controls (0/62). The movement pattern of ADHD subjects were much more persistent (1.6-fold greater b) and predictable (57% lower ApEN) than those in the contrast group. These differences were associated with very large effect sizes (2.2 - 4.7) (Figure 3). Further, there was virtually no overlap between ADHD subjects and contrast subjects on MLE (ROC Area = 1.0), and β (ROC area = 0.991) (Figure 4). Non-linear analysis revealed much stronger differences between ADHD subjects and controls than traditional linear measures. For instance, mean activity levels differed significantly between groups (F = 35.10, df = 1,121, P < 0.001, d' = 1.07, ROC = 0.857), but effect size and ROC measures were much greater for β and MLE.

ApEn, β and MLE. were significantly affected by probe dose MPH, with effect sizes measures that ranged from 1.0 - 1.2 (all p's< 0.0001; Figure 3). The percentage of ADHD subjects with evidence for a deterministic chaotic component to their head movements fell from 84% to 66% (41/62).

However, while MPH exerted strong statistical effects on these measures, they remained quite different from contrast subjects (all p's < 0.0001). This was particularly true for MLE, which was still 4.8-fold higher in ADHD after MPH (d' = 3.1). ROC analysis confirmed that discriminative differences persisted between ADHD POST and contrast subjects on β (ROC Area = 0.850) and MLE (ROC Area = 0.995). Multivariate ANOVA indicated that measure of inhibition (baseline and spike amplitude) and head marker position (β, MLE) were affected quite differently by MPH (MPH x measurement type, F = 64.49, df= 1,60, p < 0.0001). MPH produced a 58.3%±39.9% within subject reduction in composite inhibitory measures, but only a 16.6%±19.2% reduction in composite measures of positional stability.

Confirmation of a deterministic component to head position was evaluated by assessing the presence of a plateau on a dimension plot across a wide range of length scales. Altogether, 56/62 ADHD subjects PRE, 62/62 POST and 0/60 contrast subjects, showed evidence for a deterministic component to their head positions based on comparison to their surrogate data sets. The correlation dimension for the ADHD subjects was 2.46±0.37.

As seen in Table II, there was a robust correlation between baseline and spike amplitudes, but only weak correlations between spike number and measures of baseline or spike amplitudes. There were robust correlations between MLE and β (Table II). These measures correlated r ~ 0.5 with measures of baseline and spike amplitude. Principle component analysis with Varimax rotation indicated that these six activity measures segregated into three orthogonal components that explained 91.1% of the variance (Table III). The first component was strongly influenced by the three measures of positional stability (ApEn, β, MLE) and accounted for 40.2% of the variance (eigenvalue 2.4). The second component was influenced by the two measures of inhibitory amplitude and accounted for 33.5% of the variance (eignevalue 2.0), while the third component was based on the number of spikes (17.4% variance; eigenvalue 1.0).

**Table II. Cross-correlation between activity measures**

| Measures | Spike # | Spike Ampl. | Base Ampl. | App Entropy | Spectral Exp. |
|---|---|---|---|---|---|
| Spike Amplitude | 0.012 | | | | |
| Base Amplitude | -0.001 | 0.883** | | | |
| Approximate Entropy | -0.511** | -0.377** | -0.184* | | |
| Spectral Exponent | 0.399** | 0.508** | 0.460** | -0.583** | |
| Maximum Lyapunov Exponent | 0.557** | 0.507** | 0.383** | -0.836** | 0.845** |

| | | | | | |
|---|---|---|---|---|---|
| *p < 0.02, **p < 0.0001 | | | | | |

**Table III. Principle component analysis with Varimax rotation**

| | Rotated Component Matrix Components | | |
|---|---|---|---|
| Measures | 1 | 2 | 3 |
| Approximate Entropy | -0.897 | -0.059 | -0.205 |
| Spectral Exponent | 0.764 | 0.391 | 0.184 |
| Maximal Lyapunov Exponent | 0.889 | 0.283 | 0.291 |
| Basal Amplitude | 0.115 | 0.975 | 0.018 |
| Spike Amplitude | 0.319 | 0.906 | -0.068 |
| Spike Number | 0.344 | -0.054 | 0.936 |

The second component (inhibitory amplitude) correlated best with measures of performance on the cognitive control task (11 of 13 performance measures correlated to a significant degree). Some of the most significant parameters to correlate with inhibitory amplitude were: variability in response latency, errors of omission and errors of commission (Spearman's Rank Order Correlation [rs] = 0.464, 0.377, 0.317 respectively, all P < 0.001). The first component (marker stability) correlated with variability in response latency (rs = 0.225, P = 0.002) and errors of omission (rs = 0.187, P = 0.01). The third component (spike number) only correlated significantly with correct response latency (rs = 0.181, P < 0.02). Errors of commission (an index of insufficient behavioral response inhibition), correlated significantly with spike amplitude (rs = 0.361, p < 0.001) and baseline amplitude (rs = 0.321, p < 0.001), but did not correlate significantly with β or MLE.

Differences in motor activity were not due to differences in performance on the cognitive control task. First, ADHD subjects performed the task with about the same level of accuracy as controls (83.1% ± 10.6% vs 84.8% ± 11.2%; F = 0.74, df= 1,121, p > 0.3). Second, motor activity measures differed much more dramatically between ADHD subjects and controls than any of the attention measures (e.g., maximal ROC difference on attention measures = 0.694, F = 8.24, df = 1,121, p = 0.005, d' = 0.52, variability in response latency). Finally, covarying motor activity measures by the significant attention parameters did not have any discernible effect on the significance of any of the motor activity differences (e.g., MLE: F = 385.6, df=1,121, p < 0.0001; with attention covariates: F = 344.0, df= 1,118, p < 0.0001).

Finally, group differences in positional stability of the head marker were not an artifact of fatigue due to the long test session. ADHD and controls differed markedly in MLE during the first 5 minutes of the test (F = 237.5, df = 1,120, p < 0.0001; d' = 2.8, ROC = 0.970). Similarly, group differences in baseline and spike amplitude, and spike number were essentially identical whether we analyzed the first 5 minutes or the entire 15 minutes. MPH also exerted comparable effects on the first 5 minutes of the test, such that POST MPH inhibitory measures did not differ from controls, while POST MPH non-linear measures remained distinctly different than controls.

### Spike Area

The percentage, amplitude, standard deviation (SD) and coefficient of variation (COV) were calculated for spikes and baseline respectively. The spike area was calculated by multiplying the spike amplitude and the duration of the spikes. A comparison of the spike area data obtained for subjects meeting DSM-IV criteria for ADHD and normal control subjects is provided in Table IV (CTRL = normal control subjects; PRE = pre-medication ADHD subjects; and PST = post-medication ADHD subjects), showing that spike area is a measure that can be used to discriminate children with ADHD from more typical children and can be used to assess the efficacy of a therapy used to ameliorate the effects of ADHD.

**Table IV**

| **CONTROL vs. PRE** | | | | | | | | |
|---|---|---|---|---|---|---|---|---|
| | CTRL AVG | CTRL STD | CTRL N | PRE AVG | PRE STD | PRE N | F Value | P Value |
| SpArea | 42396.0034 | 17379.8033 | 82 | 100897.28 | 60946.9127 | 62 | 68.34 | 8.86E-14 |
| | | | | | | | | |

| **CONTROL vs. PST** | | | | | | | | |
|---|---|---|---|---|---|---|---|---|
| | CTRL AVG | CTRL STD | CTRL N | PST AVG | PST STD | PST N | F Value | P Value |
| SpArea | 42396.0034 | 17379.8033 | 82 | 36631.5066 | 30745.0555 | 62 | 2.02 | 0.15 |
| | | | | | | | | |

| **PRE vs. PST** | | | | | | | | |
|---|---|---|---|---|---|---|---|---|
| | PRE AVG | PRE STD | PRE N | PST AVG | PST STD | PST N | F Value | P Value |
| SpArea | 100897.28 | 60946.9127 | 62 | 36631.5066 | 30745.0555 | 62 | 67.86 | 1.71E-11 |

### Discussion

Seated activity of children with ADHD was characterized by an impaired ability to: (1) inhibit activity to low levels, (2) maintain suppression, and (3) stabilize position of the head marker. This latter problem may be a consequence of postural instability affecting head and trunk, and/or a problem with head positioning involving movements around the atlanto-occipital joint or cervical spine. Recent studies have shown that individuals with ADHD have problems with postural control while standing (Cheng et al., Beijing Da Xue Xue Bao. 39:531 (2007); and Buderath et al., Gait Posture 29:249 (2009)). However, differences between ADHD and controls in these studies were minor (Cheng et al., Beijing Da Xue Xue Bao. 39:531 (2007); and Buderath ct al., Gait Posture 29:249 (2009)), and the linear analytic methods applied were not capable of characterizing and classifying the dynamics of postural stability (Ladislao et al., Med Biol Eng Comput. 45:679 (2007); and Sasaki et al., Neurosci Res. 41:185 (2001)). Increased sway could have been an artifact of increased activity. Postural control and head positioning are characterized by strong nonlinearities due to the elastic and damping properties of muscles as well as nonlinear feedback control in the nervous system (Blaszczyk et al., Acta Neurobiol Exp (Wars) 61:105 (2001)). The head is stabilized in space by sensory inputs, vestibular and cervico-collic reflexes and the cervical musculoskeletal system (Keshner et al., J Vestib Res. 9:423 (1999)). Since these systems interact through feedback loops, and form a multi-link network, small changes in one can have remarkable and unpredictable effects throughout the network.

Between 84% and 90% of children with ADHD off medication (depending on analytical method), but none of the contrast controls, had evidence for a significant MLE in head movements. This result indicates that fluctuations in positioning of the head marker were not randomly derived, but showed sensitivity to initial conditions (i.e., slight differences in initial values can result in large or unpredictable differences, aka "butterfly effect"). To our knowledge, this is the first report showing that ADHD children have movement patterns that were deterministic and chaotic in nature.

The spectral exponent describes the persistency of movement fluctuations (Lipsitz LA. J Gerontol A Biol Sci Med Sci. 57:B115 (2002)). Elevated β suggest the presence of long-range correlations, with large amplitude movements followed by additional large amplitude movements. ApEn results revealed a significant decrease in the complexity of head marker movements in children with ADHD. We had previously reported that their head movements had a lower degree of spatial complexity, being more linear than Brownian (Teicher et al., J Am Acad Child Adolesc Psychiatry 35:334 (1996)). It appears that children with ADHD regulate head marker position using a mathematically simpler approach.

We observed these disturbances in inhibitory control and positional stability during performance of a cognitive control task. Postural sway increases with increasing demands for attention (Blanchard et al., Pediatr Phys Ther. 17:189 (2005); Olivier et al., Neuroreport. 18:817 (2007); Pellecchia GL. Gait Posture 18:29 (2003); and Schmid et al., Exp Brain Res. 179:375 (2007)). It is conceivable that enhancing postural control may, in turn, improve attentional abilities.

Probe dose of MPH significantly affected all measures in the ADHD subjects. However, while the degree and duration of inhibitory suppression fully resolved after MPH, instability in head positioning persisted. Most of these subjects continued to show evidence of deterministic chaos in their movement patterns. Rocchi (Rocchi et al., Neurosci Lett. 394:140 (2006)) reported that drugs affecting dopaminergic systems (e.g. levodopa) actually increased postural sway in patients with Parkinson's disease. Further studies are necessary to ascertain if other therapeutic strategies can fully correct these non-linear movement differences, and if doing so results in discernible clinical benefits.

This study suggests that hyperactivity and fidgeting in ADHD is a complex neurointegrative problem. It is not simply 'exuberance', or the upper end of a normal distribution (Jureidini J. J Paediatr Child Health. 32:201 (1996)). The distribution of MLE and β in ADHD children and contrast subjects were mutually distinct and essentially non-overlapping (Figure 4).

The implications of these findings suggest that elevated MLE and β may be strong biobehavioral markers for ADHD hyperactivity in children. It remains to be seen if these parameters are elevated in other disorders that enter into the differential diagnosis. We believe that deterministic chaos in head movements could be a sign of ADHD hyperactivity that normal individuals would be unable to fake.

There are several limitations of this study. First, we included only 9 to 12 years old boys, and only selected ADHD subjects who met diagnostic criteria for combined subtype. Comparable investigations needs to be conducted in girls, adolescents and adults. Further, all of the ADHD subjects in this study had received treatment with MPH. It will be important to ascertain if subjects with ADHD who have never received treatment show the same constellation of abnormalities. ADHD subjects in this study had conventional head movement measures (number of position changes, total displacement, area, and spatial complexity) that were highly comparable to those observed in ADHD subjects who had never received treatment (Teicher et al., J Am Acad Child Adolesc Psychiatry 35:334 (1996)). ADHD subjects were tested twice (PRE and POST) and controls were only tested once, raising the possibility that improvement was a practice effect. This is not the case; test-retest measures are highly concordant (unbiased estimates of reliability range from 0.92 - 0.97), and motor activity shows no improvement on placebo (Teicher et al., J Child Adolesc Psychopharmacol. 16:416 (2006)). Contrast controls were not evaluated using structured interviews, but were only screened by Teacher ratings to help exclude subjects with ADHD. Rigorously screened controls with no personal or family history of psychiatric disorders tested in the laboratory typically show even lower degrees of activity and better performance on the cognitive control task (Teicher et al., J Child Adolesc Psychopharmacol. 14:219 (2004)). Postural studies typically use force platforms to record center of pressure trajectories, though a recent study used infrared motion analysis (Miyahara et al., Hum Mov Sci. 27:705 (2008)), as we have done. It is not possible to tell in this study whether instability in head marker position resulted from postural instability or from a more specific problem in head positioning. We had previously observed that ADHD subjects differed from controls to the same degree in conventional movement measures whether markers were located on head, back, shoulder or elbow, suggesting that postural factors may be particularly important.

The application of chaos theory to complex biological systems has been widely pursued in the past few decades (Faure et al., C R Acad Sci III 324:773 (2001); Korn et al., C R Biol. 326:787 (2003); and Rossler et al., Integr Physiol Behav Sci. 29:328 (1994)). However, the interpretation of results has been the subject of much debate. This is largely due to the inherent nature of biological data, which can only provide a time series of finite length mixed with noise.

Critical issues involved in demonstrating the existence of chaotic behavior in a time series include: data size, sampling rate, existence of noise, and choice of initial parameters (Sivakumar B. Journal of Hydrology 227:1 (2000)). No accepted standard has emerged for minimal number of points, or sampling rate, as this depends largely on the underlying dynamics (Sivakumar B. Journal of Hydrology 227:1 (2000)). One previous study calculating MLE on heart rate variability used 20,000 points (Signorini et al., Proceedings IEEE-EMBS Conference, Baltimore, 1994), while another study calculating MLE, correlation dimension and Kolmogorov entropy of arterial blood pressure and cerebral blood flow velocity used 5-10 minutes of data with a sampling rate of 60 Hz (Liau et al., Med Biol Eng Comput. 46:1 (2008)). A paper calculating MLE for postural displacement used 100 seconds of data sampled at 100 Hz (Ladislao et al., Med Biol Eng Comput. 45:679 (2007)). Another study examining infants' postural development analyzed 3,840 points (Harbourne et al., Dev Psychobiol. 42:368 (2003)). Our time series with 45,000 data points collected over 15 minutes is larger than most previously published series, but collected at a slightly slower sampling rate (50 Hz). Nevertheless, this series should be quite adequate for calculating MLE.

Noise is another critical concern. A previous study pointed out that low pass filtering can introduce spurious Lyapunov exponents (Badii et al., Phys Rev Lett. 60:979 (1988)). Hence, MLE was analyzed following non-linear filtering of the original data set. The appropriate choices of delay time and embedding dimension parameters are critical. We used the mutual information (Fraser et al., Phys Rev A. 33:1134 (1986)), and false nearest neighbor (Kennel et al., Phys Rev A. 45:3403 (1992)) methods to derive these parameters. These seem to have been reasonable choices, given the evidence for statistically significant MLEs in the surrogate time series. Results were also verified using the correlation dimension method (Sivakumar B. Journal of Hydrology 227:1 (2000)).

These findings suggest that hyperactivity observed in ADHD is a complex phenomenon that appears to stem from deficits in regulatory systems that differ in degree of correctability by MPH.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features hereinbefore set forth, and follows in the scope of the claims.

Other embodiments are within the claims.

## Claims

1. A system for diagnosing a disorder selected from ADHD and related disorders in a subject comprising:
(i) an input component configured to receive information comprising motor activity data obtained by monitoring the movements of said subject; and
(ii) a processor provided with a computer program for calculating a value for a measure of inhibitory control in said subject, **characterized in that** the measure of inhibitory control is the spike amplitude or the baseline amplitude of said motor activity data.

2. The system of claim 1, wherein said measure of inhibitory control is the spike amplitude of said motor activity data.

3. The system of claim 1, wherein said measure of inhibitory control is the baseline amplitude of said motor activity data.

4. The system of claim 1, wherein said disorder is ADD, ADHD, or
Hyperkinetic Disorder, or a specific subtype of ADD, ADHD, or Hyperkinetic Disorder.

5. A method of diagnosing ADHD or a related disorder in a subject comprising the steps of:
(i) providing motor activity data having been collected by using a motion analysis device to record movements of said subject;
(ii) analyzing said motor activity data to calculate a value for a measure of inhibitory control in said subject using a computer program provided on a processor; and
(iii) on the basis of said value, diagnosing ADHD or a related disorder in said subject:
**characterized in that** the measure of inhibitory control is the spike amplitude or baseline amplitude of said motor activity.

6. The method of claim 5, wherein said measure of inhibitory control is the spike amplitude of said motor activity.

7. The method of claim 5, wherein said measure of inhibitory control is the baseline amplitude of said motor activity.

8. The method of claim 5, wherein said disorder is ADD, ADHD, or
Hyperkinetic Disorder, or a specific subtype of ADD, ADHD, or Hyperkinetic Disorder.

9. Use of the system of claim 1.

## Patentansprüche

1. System zum Diagnostizieren einer Erkrankung, die aus ADHD und verwandten Erkrankungen ausgewählt ist, bei einer Person, wobei das System Folgendes umfasst:
(i) eine Eingabekomponente, die dazu konfiguriert ist, Informationen zu empfangen, die Motorikdaten umfassen, die durch Überwachen der Bewegungen der Person erhalten wurden; und
(ii) einen Prozessor, der mit einem Computerprogramm zum Berechnen eines Werts für eine Maßgröße der inhibitorischen Kontrolle in der Person versehen ist, **dadurch gekennzeichnet, dass** die Maßgröße der inhibitorischen Kontrolle die Spike-Amplitude oder die Baseline-Amplitude der Motorikdaten ist.

2. System nach Anspruch 1, wobei die Maßgröße der inhibitorischen Kontrolle die Spike-Amplitude der Motorikdaten ist.

3. System nach Anspruch 1, wobei die Maßgröße der inhibitorischen Kontrolle die Baseline-Amplitude der Motorikdaten ist.

4. System nach Anspruch 1, wobei die Erkrankung ADD, ADHD oder eine hyperkinetische Störung oder ein spezifischer Subtyp von ADD, ADHD oder einer hyperkinetischen Störung ist.

5. Verfahren zum Diagnostizieren von ADHD oder einer verwandten Erkrankung bei einer Person, wobei das Verfahren die folgenden Schritte umfasst:
(i) Bereitstellen von Motorikdaten, die durch Verwendung einer Bewegungsanalysevorrichtung, um Bewegungen der Person aufzuzeichnen, gesammelt wurden;
(ii) Analysieren der Motorikdaten, um einen Wert für eine Maßgröße der inhibitorischen Kontrolle in der Person unter Verwendung eines Computerprogramms, das auf einem Prozessor vorgesehen ist, zu berechnen; und
(iii) auf der Basis des Werts Diagnostizieren von ADHD oder einer verwandten Erkrankung bei der Person;
**dadurch gekennzeichnet, dass** die Maßgröße der inhibitorischen Kontrolle die Spike-Amplitude oder Baseline-Amplitude der Motorikdaten ist.

6. Verfahren nach Anspruch 5, wobei die Maßgröße der inhibitorischen Kontrolle die Spike-Amplitude der Motorikdaten ist.

7. Verfahren nach Anspruch 5, wobei die Maßgröße der inhibitorischen Kontrolle die Baseline-Amplitude der Motorikdaten ist.

8. Verfahren nach Anspruch 5, wobei die Erkrankung ADD, ADHD oder eine hyperkinetische Störung oder ein spezifischer Subtyp von ADD, ADHD oder einer hyperkinetischen Störung ist.

9. Verwendung des Systems nach Anspruch 1.

## Revendications

1. Système pour diagnostiquer un trouble sélectionné parmi TDAH et des troubles connexes chez un sujet comprenant :
(i) un composant d'entrée configuré pour recevoir des informations comprenant des données d'activité motrice obtenues en surveillant les mouvements dudit sujet ; et
(ii) un processeur pourvu d'un programme d'ordinateur pour calculer une valeur pour une mesure du contrôle inhibiteur chez ledit sujet, **caractérisé en ce que** la mesure du contrôle inhibiteur est l'amplitude de pointe ou l'amplitude de base desdites données d'activité motrice.

2. Système selon la revendication 1, dans lequel ladite mesure du contrôle inhibiteur est l'amplitude de pointe desdites données d'activité motrice.

3. Système selon la revendication 1, dans lequel ladite mesure du contrôle inhibiteur est l'amplitude de base desdites données d'activité motrice.

4. Système selon la revendication 1, dans lequel ledit trouble est TDA, TDAH, ou un trouble hyperkinétique, ou un sous-type spécifique de TDA, de TDAH, ou de trouble hyperkinétique.

5. Procédé de diagnostic de TDAH ou d'un trouble connexe chez un sujet, comprenant les étapes de :
(i) la fourniture de données d'activité motrice ayant été collectées en utilisant un dispositif d'analyse de mouvements pour enregistrer des mouvements dudit sujet ;
(ii) l'analyse desdites données d'activité motrice pour calculer une valeur pour une mesure du contrôle inhibiteur chez ledit sujet en utilisant un programme d'ordinateur prévu sur un processeur ; et
(iii) en fonction de ladite valeur, le diagnostic de TDAH ou d'un trouble connexe chez ledit sujet ;
**caractérisé en ce que** la mesure du contrôle inhibiteur est l'amplitude de pointe ou l'amplitude de base de ladite activité motrice.

6. Procédé selon la revendication 5, dans lequel ladite mesure du contrôle inhibiteur est l'amplitude de pointe de ladite activité motrice.

7. Procédé selon la revendication 5, dans lequel ladite mesure du contrôle inhibiteur est l'amplitude de base de ladite activité motrice.

8. Procédé selon la revendication 5, dans lequel ledit trouble est TDA, TDAH, ou un trouble hyperkinétique, ou un sous-type spécifique de TDA, de TDAH, ou de trouble hyperkinétique.

9. Utilisation du système selon la revendication 1.
